# EUROPEAN PATENT APPLICATION

(11) **EP 2 090 306 A1**
(43) Date of publication of application: **19.08.2009**
(21) Application number: 08002657.8
(22) Date of filing: 13.02.2008
(51) Int. Cl.: A61K 31/403, A61K 31/404, C07D 403/06, A61P 35/00

(54) **Pharmaceutical compositions comprising N-[2-(diethylamino)ethyl]-5-[(5-fluoro-1,2-dihydro-2-oxo-3H-indol-3-ylidene)methyl]-2,4-dimethyl-1H-pyrrole-3-carboxamide**

(71) Applicant: Ratiopharm GmbH, 89079 Ulm (DE)
(72) Inventor: Pätz, Jana, 86424 Dinkelscherben (DE); Muskulus, Frank, 82194 Gröbenzell (DE)
(74) Representative: Teipel, Stephan

(57) **Abstract**

The present invention relates to a pharmaceutical composition comprising N-[2-(di-ethylamino)ethyl]-5-[(5-fluoro-1,2-dihydro-2-oxo-3H-indol-3-ylidene)methyl]-2,4-dimethyl-1H-pyrrole-3-carboxamide or a pharmaceutically acceptable salt thereof as active pharmaceutical ingredient, wherein the active pharmaceutical ingredient is present in amorphous or partially amorphous form.

## Description

The invention relates to a pharmaceutical composition comprising N-[2-(diethylamino)ethyl]-5-[(5-fluoro-1,2-dihydro-2-oxo-3H-indol-3-ylidene)methyl]-2,4-dimethyl-1H-pyrrole-3-carboxamide and a process of preparing such composition.

The compound N-[2-(diethylamino)ethyl]-5-[(5-fluoro-1,2-dihydro-2-oxo-3H-indol-3-ylidene)methyl]-2,4-dimethyl-1H-pyrrole-3-carboxamide (herein also referred to as compound I) is a receptor tyrosine kinase inhibitor which is used to treat disorders like renal cell carcinoma (RCC) and gastrointestinal stromal tumor (GIST). Its activity relies on the inhibition of cellular signaling by targeting multiple receptor tyrosine kinases including platelet-derived growth factor receptors and vascular endothelial growth factor receptors. Since both kinds of receptors are involved in tumor angiogenesis and tumor cell proliferation, the simultaneous inhibition of these targets results in both reduced tumor vascularization and cancer cell death. These effects are responsible for the finally observed shrinkage of the renal cell carcinoma and gastrointestinal stromal tumor, respectively.

Compound I and its pharmaceutical effects on disorders like cancer are described in WO 01/060814. Further medical uses of compound I and its salts are inter alia known from WO 01/045689 and WO 03/035009.

WO 01/060814 discloses two processes of preparing compound I. According to these and other known manufacturing processes, compound I is obtained as a solid. One of the forms of compound I is its crystalline malic acid salt as described in WO 03/016305. The solid exhibits poor solubility in water affecting its oral bioavailability. Moreover, it has been reported that it causes manufacturing problems when being processed by higher concentrations, especially concentrations over 40 wt.% (see for example WO 04/024127).

Typically, compound I is administered in a dose of 50 mg once daily, which, if necessary, has to be varied according to individual tolerance and safety. Thus, in order to obtain sufficiently flexible dosing, individual dosage forms generally contain 12.5, 25 and 50 mg of compound I. Capsules comprising the malate salt of compound I are sold under the brand name Sutent® (by Pfizer Pharma). In these capsules the problem of the compound's poor solubility is overcome by the addition of sodium dodecyle sulphate (SDS), which promotes the dissolution of compound I from the pharmaceutical composition. Still, since the surfactant SDS can cause health problems (possible irration of the gastrointestinal tract when orally administered; hazardous when being inhaled - relevant for personnel involved with the formulation process; possibly harmfull to the oral mucosa; risk of dermatitis when in contact with the skin; see also Seth, P.L.; Münzel, K.; Pharm Ind. 20, 50 (1958) and Rosenthaler, L.; Chem. Rundschau 10, 201 (1957)) and might impair the manufacturing process (e.g. foaming during the wet granulation process), this is not an optimal solution to the problem.

Moreover, good homogeneity and flowability of a pharmaceutical composition are a prerequisite for a successful manufacture of for example tablets and capsules on a production scale. The above-mentioned manufacturing problems can attribute to the crystalline form of compound I.

It is therefore an object of the invention to provide a pharmaceutical composition comprising compound I or a pharmaceutically acceptable salt thereof which does not encounter the above problems. In particular the composition should possess improved properties like solubility, homogeneity and flowability.

It has now been found that the above problems can be overcome by providing a pharmaceutical composition comprising compound I or a salt of it in amorphous form instead of crystalline form.

Thus, the present invention relates to a pharmaceutical composition comprising N-[2-(di-ethylamino)ethyl]-5-[(5-fluoro-1,2-dihydro-2-oxo-3H-indol-3-ylidene)methyl]-2,4-dimethyl-1H-pyrrole-3-carboxamide or a pharmaceutically acceptable salt thereof as active pharmaceutical ingredient, wherein the active pharmaceutical ingredient is present in amorphous or partially amorphous form.

N-[2-(diethylamino)ethyl]-5-[(5-fluoro-1,2-dihydro-2-oxo-3H-indol-3-ylidene)methyl]-2,4-dimethyl-1H-pyrrole-3-carboxamide (compound I) has the following chemical structure:

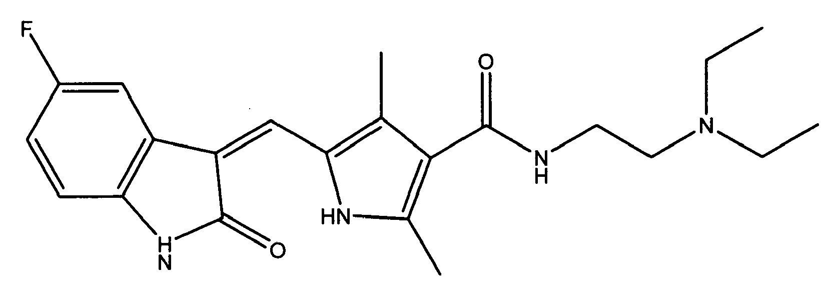

Compound I as well as its salts can be readily synthesized using techniques well known in the art. Syntheses of compound I are disclosed for example in WO 01/060814.

The term "amorphous form" refers to a form of active pharmaceutical ingredient which has no long-range order like crystalline forms. The atoms of a material present in amorphous form exist in a non-uniform array. It is for example possible to distinguish amorphous from crystalline forms of a compound by powder X-ray diffraction.

The term "active pharmaceutical ingredient " (API) refers to compound I in its salt free or salt form. Thus, if a pharmaceutically acceptable salt of compound I is employed, active pharmaceutical ingredient refers to compound I including the salt component.

The term "partially amorphous form" refers to a form of the active pharmaceutical ingredient comprising aside from a portion of it in amorphous form a portion in crystalline form. Preferably, at least 10 wt.%, more preferably at least 20 wt.%, at least 30 wt.%, at least 40 wt.%, at least 50 wt.% of the active pharmaceutical ingredient are in amorphous form, even more preferably at least 90 wt.%, most preferably at least 98 wt.%, wherein the respective amounts being referred to the total weight of the active pharmaceutical ingredient in the composition of the present invention.

The term "pharmaceutical composition" refers to single dosage forms, such as tablets, capsules, pellets, etc., as well as powders or granules which are used in the preparation of single dosage forms. Where it is referred to the total weight of the pharmaceutical composition and the pharmaceutical composition in a single dosage form the total weight is the weight of the single dosage form excluding, if applicable, the weight of any coating or capsule shell.

The active pharmaceutical ingredient is preferably present in the pharmaceutical composition in an amount of more than 40 wt.%, more preferably at least 53 wt.% and even more preferably at least 70 wt.%, wherein the respective amounts being referred to the weight of the total composition. Further preferred portions of the active pharmaceutical ingredient in the pharmaceutical composition are more than 40 wt.%, more than 41 wt.%, more than 42 wt.%, more than 43 wt.%, more than 44 wt.%. more than 45 wt.%, more than 50 wt.%, more than 53 wt.%, more than 60 wt.% or more than 70 wt.%, wherein the respective amounts are being referred to the weight of the total composition.

Due to the amorphous form of the active pharmaceutical ingredient, the pharmaceutical composition of the present invention possesses excellent solubility, homogeneity and flowability. Further, it shows a great workability even if the portion of active pharmaceutical ingredient in the composition is higher than 40 wt.%.

Even more advantageous properties regarding solubility, homogeneity and flowability can be achieved if the pharmaceutical composition of the present invention has a mean particle size of 0.3 to 300 µm, preferably 5 to 300 µm.

A bulk density of the pharmaceutical composition ranging from of 0.2 to 0.8 g/ml, preferably of 0.25 to 0.7 g/ml, more preferably of 0.3 to 0.6 g/ml is advantageous.

The pharmaceutical composition of the invention preferably possesses Hausner ratios in the range of 1.1 to 1.6, preferably of 1.2 to 1.5. The Hausner factor is the ratio of bulk density to tapped density.

The pharmaceutical composition of the present invention may further comprise one or more pharmaceutically acceptable excipients, such as fillers, binding agents, lubricants, glidants, antisticking agents, crystallization inhibitors and disintegrating agents. As pharmaceutically acceptable excipients conventional excipients known to the person skilled in the art may be used. See for example "Lexikon der Hilfsstoffe für Pharmazie, Kosmetik und angrenzende Gebiete", edited by H. P. Fiedler, 4th Edition, Edito Cantor, Aulendorf and earlier editions, and "Handbook of Pharmaceutical Excipients", Third Edition, edited by Arthur H. Kibbe, American Pharmaceutical Association, Washington, USA, and Pharmaceutical Press, London.

Preferred examples of the fillers are lactose, mannitol, sorbitol or microcrystalline cellulose. The filler is suitably present in an amount of 0 to 80 wt.%, preferably of 10 to 20 wt.% of the total weight of the composition.

The binding agent can for example be microcrystalline cellulose (MCC) or hydroxypropylmethyl cellulose (HPMC). Preferably the binding agent is present in an amount of 1 to 25 wt.%, more preferably at 2 to 10 wt.% of the total weight of the composition.

The lubricant is preferably a stearate, more preferably an earth alkali metal stearate, such as magnesium stearate. The lubricant is suitably present in an amount of 0.1 to 2 wt.%, preferably about 1 wt.% of the total weight of the composition.

Preferred crystallization inhibitors may be selected from the group consisting of polyvinylpyrrolidone (PVP); organic acids, e.g. citric acid; inorganic salts, e.g. ammonium carbonate; and methacrylates. The crystallization inhibitor is suitably present in an amount of 0.1 to 10 wt.%, preferably 2 to 5 wt.% of the total weight of the composition.

Preferred disintegrating agents are croscarmellose sodium, sodium carboxymethyl starch or cross-linked polyvinylpyrrolidone (crospovidone). The disintegrating agent is suitably present in an amount of 0.1 to 20 wt.%, more preferably at about 0.5 to 7 wt.% of the total weight of the composition.

The glidant can for example be colloidal silicon dioxide. Preferably the binding agent is present in an amount of 0.5 to 8 wt.%, more preferably at 0.5 to 3 wt.% of the total weight of the composition.

The antisticking agent is for example talcum and may be present in amounts of 1 to 5 %.wt, more preferably in an amount of 1.5 to 3 wt.% of the total weight of the composition.

A person skilled in the art may use these or other excipients in regard to the selected process of preparing the pharmaceutical composition of the invention.

The pharmaceutical composition of the present invention can be formulated in any known matter, preferably as tablets, capsules, granules, pellets or sachets. A particularly preferred pharmaceutical composition is in the form of capsules. The pharmaceutical composition may contain dosage amounts of 12.5, 25 and 50 mg of the active pharmaceutical ingredient. Thus the administered amount can be readily varied according to individual tolerance and safety warranting more flexible dosing than the standard dose of 50 mg once daily.

A further aspect of the present invention provides a process of preparing a pharmaceutical composition comprising compound I or a pharmaceutically acceptable salt thereof as active pharmaceutical ingredient wherein the active pharmaceutical ingredient is present in amorphous or partially amorphous form. The process comprises the following steps:
(a) providing compound I or a pharmaceutically acceptable salt thereof as active pharmaceutical ingredient and optionally at least one pharmaceutically acceptable excipient; and
(b) transferring the active pharmaceutical ingredient in its amorphous or partially amorphous form.

In a first embodiment of this process the active pharmaceutical ingredient is transferred in step (b) in its amorphous or partially amorphous form by spray drying in the presence or absence of any excipient(s). This leads to an enhanced particle surface porosity and, if at least one excipient is present, an optimized embedding of the active pharmaceutical ingredient into the excipient(s), thereby improving solubility, homogeneity and flowability of the composition of the invention.

Preferably, the spray drying is performed in the presence of one of the following excipients: polymers based on cellulose, polyvinylpyrrolidone (PVP) or vinylpyrrolidone-vinyl acetate copolymers. More preferably, a combination of at least one insoluble and at least one soluble excipient is used. Here, "soluble" and "insoluble" refers to the solubility of the excipient in the solvent used in the spray drying step.

In this context PVP also serves as crystallization inhibitor and thus stabilizes the amorphous or partially amorphous form into which compound I passes during the spray-drying process. Other possible crystallization inhibitors are e.g. organic acids, e.g. citric acid; inorganic salts, e.g. ammonium carbonate; and methacrylates, preferably methacrylates with low glass transition temperatures. Depending on the excipients and the process parameters, mean particle sizes of 5 to 300 µm and bulk densities of 0.3 to 0.7 g/ml are preferably obtained. The ratio between tapped density and bulk density is strongly dependent on the choice of excipients, and preferred granulates according to this embodiment of the present invention have Hausner ratios between 1.2 and 1.5.

In a second embodiment of the process of the invention, the active pharmaceutical ingredient is transferred in its amorphous or partially amorphous form in above step (b) by lyophilizing, i.e. the solvent is evaporated from a solution containing the active pharmaceutical ingredient and optionally one or more excipients via freeze-drying under vacuum. This leads to an enhanced particle surface porosity and, if at least one excipient is present, an optimized embedding of the active pharmaceutical ingredient into the excipient(s), thereby improving solubility, homogeneity and flowability.

Preferably, mixtures of soluble and insoluble excipients in any ratio are employed. Here, "soluble" and "insoluble" refers to the solubility of the excipient in the solvent used in the lyophilizing step. Application of this embodiment results in an amorphization or partial amorphization of the active pharmaceutical ingredient. Depending on the excipients, the drying parameters and, if applicable, the milling technology, mean particle sizes of 0.3 to 300 µm and bulk densities between 0.25 and 0.6 g/ml are preferably obtained. Preferred granulates according to this embodiment of the present invention have Hausner ratios between 1.2 and 1.5.

The invention is now illustrated in the following examples which are not to be constructed as being limiting.

### Examples

### Example 1: Lyophilizing of Compound I

The following procedure was used to prepare the pharmaceutical composition having the formulation listed in Table 1 and comprising a solid amorphous form of compound I.

Compound I and mannitol were lyophilized from an aqueous-alcoholic solution (pH 7.4) at 20 to 100 mbar and -40 to -60 °C and dried at 1013 mbar and 25 to 45 °C. The lyophilisate was mixed with sodium croscarmellose and magnesium stearate and filled into hard gelatin capsules (size 4).

**Table 1**

| Ingredient | Product | Function | Amount |
|---|---|---|---|
| Compound I | micronized | API | 25 mg |
| Mannitol | Mannit | filler | 18.6 mg |
| Sodium croscarmellose | Ac-Di-SOL | disintegrant | 2.5 mg |
| Magnesium stearate | tbd | antisticking | 0.5 mg |
| Total | | | 46.6 mg |

### Example 2: Spray-drying of Compound I

The following procedure was used to prepare the pharmaceutical composition having the formulation listed in Table 2 and comprising a solid amorphous form of compound I.

Microcrystalline cellulose (MCC) was suspended in an aqueous solution of compound I and povidone (5 to 50 % combined dry weight). This suspension was spray dried. Then, sodium croscarmellose, colloidal silicon dioxide and magnesium stearate were added and mixed with the granules obtained in the spray drying process. The mixture was filled into hard gelatin capsules (size 4).

**Table 2**

| Ingredient | Product | Function | Amount |
|---|---|---|---|
| Compound I | | API | 25 mg |
| MCC | Avicel PH 101 | filler | 10.0 mg |
| Povidone | Kollidon 25 | binder | 8.0 mg |
| Sodium croscarmellose | Ac-Di-SOL | disintegrant | 2.8 mg |
| Colloidal silicon dioxide | Aerosil | glidant | 0.2 mg |
| Magnesium stearate | tbd | antisticking | 0.6 mg |
| Total | | | 46.6 mg |

## Claims

1. Pharmaceutical composition comprising N-[2-(diethylamino)ethyl]-5-[(5-fluoro-1,2-dihydro-2-oxo-3H-indol-3-ylidene)methyl]-2,4-dimethyl-1H-pyrrole-3-carboxamide or a pharmaceutically acceptable salt thereof as active pharmaceutical ingredient, wherein the active pharmaceutical ingredient is present in amorphous or partially amorphous form.

2. The pharmaceutical composition according to claim 1, comprising the active pharmaceutical ingredient in an amount of more than 40% by weight, preferably of more than 53% by weight of the total weight of the composition.

3. The pharmaceutical composition according to any of the preceding claims, having a mean particle size of 0.3 to 300 µm.

4. The pharmaceutical composition according to any of the preceding claims, having a bulk density of 0.2 to 0.8 µ/ml.

5. The pharmaceutical composition according to any of the preceding claims, having a Hausner ratio of 1.1 to 1.6.

6. The pharmaceutical composition according to any of the preceding claims, further comprising one or more pharmaceutically acceptable excipients, such as fillers, binding agents, lubricants, glidants, crystallization inhibitors and disintegrating agents.

7. Process of preparing a pharmaceutical composition according to any of claims 1 to 6, comprising
(a) providing N-[2-(diethylamino)ethyl]-5-[(5-fluoro-1,2-dihydro-2-oxo-3H-indol-3-ylidene)methyl]-2,4-dimethyl-1H-pyrrole-3-carboxamide or a pharmaceutically acceptable salt thereof as active pharmaceutical ingredient and optionally at least one pharmaceutically acceptable excipient; and
(b) transferring the active pharmaceutical ingredient into its amorphous or partially amorphous form.

8. The process according to claim 7, wherein in step (b) the active pharmaceutical ingredient is transferred into its amorphous or partially amorphous form by spray drying.

9. The process according to claim 8, wherein the spray drying is performed in the presence of at least one soluble and at least one insoluble excipient.

10. The process according to claim 9, wherein said soluble excipient is polyvinylpyrrolidone or a vinylpyrrolidone-vinyl acetate copolymer and said insoluble excipient is a polymer based on cellulose.

11. The process according to claim 7, wherein in step (b) the active pharmaceutical ingredient is transferred in its amorphous or partially amorphous form by lyophilization.

12. The process according to claim 11, wherein the active pharmaceutical ingredient is lyophilized in the presence of at least one soluble and at least one insoluble excipient.

13. N-[2-(diethylamino)ethyl]-5-[(5-fluoro-1,2-dihydro-2-oxo-3H-indol-3-ylidene)methyl]-2,4-dimethyl-1H-pyrrole-3-carboxamide or a pharmaceutically acceptable salt thereof in amorphous or partially amorphous form.

14. Use of the compound of claim 13 for the preparation of a pharmaceutical composition.
